# EUROPÄISCHE PATENTSCHRIFT

(11) **EP 0 926 498 B1**
(45) Veröffentlichungstag und Bekanntmachung des Hinweises auf die Patenterteilung: **01.10.2008**
(21) Anmeldenummer: 98124287.8
(22) Anmeldetag: 21.12.1998
(51) Int. Cl.: G01N 33/558, G01N 33/566, G01N 33/553, G01N 33/569, C12Q 1/68

(54) **Verfahren zur Analytbestimmung unter Verwendung eines universellen Analysenelementes**
Method for determining an analyte using a universal test strip
Procédé pour la détermination des analytes utilisant un élément d'analyse universel

(30) Priorität: 24.12.1997 DE 19757980; 15.04.1998 DE 19816550
(43) Veröffentlichungstag der Anmeldung: 30.06.1999
(60) Teilanmeldung: 08009295.0
(73) Patentinhaber: Roche Diagnostics GmbH, 68305 Mannheim (DE)
(72) Erfinder: Klepp, Jürgen, Dr., 76131 Karlsruhe (DE); Hiller, Gerhard, Dr., 68305 Mannheim (DE); Nichtl, Alfons, Dr., 82383 Hohenpeissenberg (DE); Fischer, Thomas, 69231 Rauenberg (DE); Hösch, Martina, 68163 Mannheim (DE)

(56) Entgegenhaltungen:
- EP-A1- 0 186 799
- EP-A1- 0 291 194
- DE-A1- 19 609 838
- US-A- 5 141 850
- US-A- 5 591 645

## Beschreibung

Die Erfindung betrifft ein Verfahren zur Bestimmung eines Analyts unter Verwendung eines Analysenelementes enthaltend in oder auf Material, das einen Flüssigkeitstransport zwischen Zonen ermöglicht, eine Probenauftragszone und eine hierzu flußabwärts gelegene Nachweiszone, wobei die Nachweiszone einen Partner 1 eines spezifischen Bindungspaares 1 so immobilisiert enthält, daß er in der Lage ist, mit Partner 2 des spezifischen Bindungspaares 1, der nicht der Analyt ist, eine Bindung einzugehen, wenn dieser ihn kontaktiert sowie ein Verfahren zur Bestimmung eines Analyts mittels spezifischer Bindungspaare. Es wird auch einen Kit zur Bestimmung eines Analyts enthaltend ein Analyseelement beschrieben.

Aus dem Stand der Technik sind Analysenelemente bekannt, die die zur Bestimmung eines Analyts erforderlichen Reagenzien in oder auf Trägermaterialien vorliegen haben. Als Beispiele seien hier genannt: US-Patent 4,861,711, US-Patent 5,591,645 und EP-A-0 291 194. Den in diesen Schriften beschriebenen Analysenelementen ist gemeinsam, daß sie speziell für die Durchführung immunologischer Nachweisverfahren geeignet sind. Sie beinhalten eine Probenauftragszone und eine hierzu flußabwärts gelegene Nachweiszone. Eine flüssige Probe durchwandert aufgrund von Kapillarkräften innerhalb eines porösen Trägermaterials verschiedene Zonen zwischen Probenaufgabe- und Nachweiszone und nimmt dabei die für den Nachweis des Analyts erforderlichen Reagenzien auf und bringt sie mit dem Analyt in der Probe zur Reaktion.

In der Nachweiszone ist ein Bindungspartner immobilisiert, der in der Lage ist, spezifisch den zu bestimmenden Analyt zu binden. Dies erfordert, daß bei unterschiedlichen Analyten unterschiedliche Bindungspartner für den Analyt auf einer Festphase immobilisiert werden müssen.

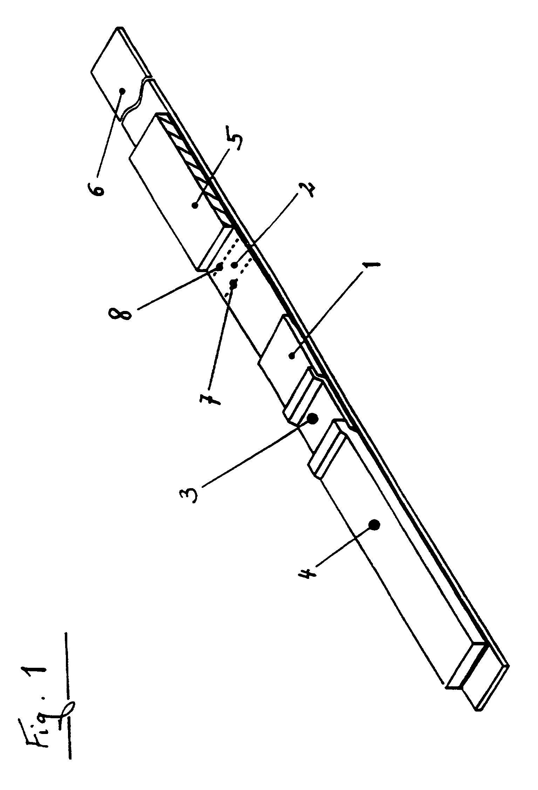

Aus US-Patent 4,861,711 ist aus Figur 1 in Verbindung mit der Beschreibung, beispielsweise in Spalte 5, Zeile 57 bis Spalte 6, Zeile 48 auch bekannt, in der Nachweiszone einen Partner 1 eines spezifischen Bindungspaares 1 zu immobilisieren, der nicht den Analyt bindet, sondern dort universell einsetzbar ist, weil er ein Epitop als Partner 2 des spezifischen Bindungspaares 1 bindet, das auf einer Substanz vorhanden ist, die den Analyt spezifisch bindet. So wird der bewegliche Komplex aus Analyt und diesen bindender Substanz im Verlauf der Nachweisreaktion in der Nachweiszone fixiert und von unkomplexierten beweglichen Reaktionskomponenten abgetrennt.

US5141850 beschreibt einen Teststreifen umfassend drei Zonen. Eine davon ist eine Aufgabezone für die Probe, eine ist mit einem Analyt-spezifischen Goldmarkierten Antikörper imprägniert, und die dritte ist mit einem Analyt-spezifischen Biotin-markierten Antikörper imprägniert. Die Probe bewegt sich entlang des Teststreifens und bildet einen Sandwich-Komplex mit den zwei markierten Antikörpern. Der Komplex wurde in der Nachweiszone gefangen mittels einen immobilisierten Latex-Avidin-Komplex und wurde nachgewiesen.

Eine markierte, für den Analyt spezifische Substanz spielt eine sehr wesentliche Rolle, weil nur deren Bindung an den Analyt und die spätere Immobilisierung des gebildeten Komplexes aus Analyt und gegen den Analyt gerichtete markierte Substanz in der Nachweiszone sowie das Entfernen der beweglichen unreagierten Reaktionskomponenten aus der Nachweiszone das Vorliegen von Analyt in der flüssigen Probe zu zeigen vermag. Die aus dem Stand der Technik bekannten markierten Substanzen sind für den Analyt spezifisch, das heißt, im Falle von Sandwich-Assays handelt es sich um markierte Substanzen, wie Antikörper oder Antigene, die spezifisch mit dem zu bestimmenden Analyt (Antigen oder Antikörper) reagieren. Dies erfordert jedoch, daß je nach Analyt unterschiedliche markierte, spezifische Bindungspartner für den Analyt hergestellt werden müssen.

Als Markierung ist eine Vielzahl von Substanzen aus dem Stand der Technik bekannt. Während früher radioaktive Markierungen mit allen ihren Nachteilen benutzt wurden, wurden diese Markierungen später vor allem durch Enzymmarkierungen abgelöst. Heute werden in Analysenelementen, wie sie in den zuvor beschriebenen Schriften des Standes der Technik beschrieben sind, vor allem partikuläre Markierungen, insbesondere Gold- oder Latexpartikel eingesetzt. Die Herstellung von Konjugat aus Markierung und spezifisch an den Analyt bindender Substanz ist aufwendig und muß, wenn unterschiedliche Analyten bestimmt werden sollen, für jeden einzelnen analytspezifischen Bindungspartner optimiert werden. Außerdem muß bei Analysenelementen das Material, auf dem dieses Konjugat vorliegt und transportiert wird, optimal den Erfordernissen von Fall zu Fall angepaßt werden. Vor allem Stabilitätsprobleme sind hierbei oft zu lösen.

Aufgabe der vorliegenden Erfindung war es deshalb, einen universell verwendbaren Aufbau eines Analysenelementes zur Verfügung zu stellen, der unabhängig von dem zu bestimmenden Analyt immer verwendet werden kann, sofern dieser Analyt oder eine vom Analyt abgeleitete und diesen repräsentierende Substanz durch spezifische Paarbindung nachweisbar ist.

Diese Aufgabe wird durch das Verfahren der Erfindung gelöst, wie er in den Ansprüchen dargestellt ist.

Beschrieben wird ein Analyseelement zur Bestimmung eines Analyts enthaltend in oder auf Material, das einen Flüssigkeitstransport zwischen Zonen ermöglicht, eine Probenauftragszone und eine hierzu flußabwärts gelegene Nachweiszone, wobei die Nachweiszone Partner 1 eines spezifischen Bindungspaares 1 so immobilisiert enthält, daß er in der Lage ist, mit Partner 2 des spezifischen Bindungspaares 1, der nicht Analyt ist, eine Bindung einzugehen, wenn er ihn kontaktiert, dadurch gekennzeichnet, daß flußaufwärts der Nachweiszone ein markierter Partner 1 eines spezifischen Bindungspaares 2 auf einem Material durch Flüssigkeit ablösbar imprägniert vorliegt und in der Lage ist, mit Partner 2 des spezifischen Bindungspaares 2, der nicht der Analyt ist, eine Bindung einzugehen, wenn dieser ihn kontaktiert, wobei Partner 2 des spezifischen Bindungspaares 1 und Partner 2 des spezifischen Bindungspaares 2 spezifisch an den zu bestimmenden Analyt gebunden oder durch Reaktion unter Beteiligung des zu bestimmenden Analyts Teile einer von vom Analyt abgeleiteten und diesen repräsentierenden Substanz sind.

Beschrieben wird auch ein Kit zur Bestimmung eines Analyts enthaltend ein Analyseelement, wie es vorstehend charakterisiert worden ist, sowie weiter enthaltend mindestens einen Partner aus der Gruppe Partner 2 des spezifischen Bindungspaares 1 und Partner 2 des spezifischen Bindungspaares 2.

Gegenstand der Erfindung ist ein Verfahren zur Bestimmung eines Analyts mittels spezifischer Bindungspaare dadurch gekennzeichnet, daß eine von Analyt abgeleitete und diesen repräsentierende Substanz, die Partner 2 eines spezifischen Bindungspaares 1 und Partner 2 eines spezifischen Bindungspaares 2 beinhaltet in einem Analyseelement zur Bestimmung eines Analyts mit markiertem Partner des spezifischen Bindungspaares 2 in Kontakt gebracht, durch Flüssigkeitstransport in dem Analysenelement in Richtung der flußabwärts der Probenauftragszone gelegenen Nachweiszone bewegt, in der Nachweiszone an Partner 1 des spezifischen Bindungspaares 1 gebunden und anhand der Markierung des Partners 1 des spezifischen Bindungspaares 2 bestimmt wird.

Wesentlich für das Analysenelement ist, daß sich Flüssigkeit innerhalb des Analysenelementes in Richtung auf die Nachweiszone bewegen kann. Ein solcher Flüssigkeitsfluß ist beispielsweise in einem entsprechend hergerichteten Hohlkörper durch Schwerkraft möglich. Vorrichtungen, die einen Flüssigkeitstransport durch Zentrifugalkraft als einer Form der Schwerkraft ermöglichen, sind beispielsweise aus EP-B 0 052 769 bekannt. Bevorzugt enthalten Analysenelemente jedoch saugfähige Materialien, die Flüssigkeit durch Kapillarkraft zu bewegen vermögen. Die Materialien der einzelnen Zonen des Analysenelementes können hierbei gleich oder auch verschieden sein. Häufig wird es so sein, daß unterschiedliche Zonen aus unterschiedlichen Materialien bestehen, wenn diese optimal ihre Aufgabe erfüllen sollen.

Als mögliche saugfähige, kapillaraktive Materialien kommen grundsätzlich alle solche in Frage, die generell in sogenannten "Trockentesten", wie sie beispielsweise in US-A 4,861,711, US-A 5,591,645 oder EP-A-0 291 194 beschrieben sind, zur Flüssigkeitsaufnahme eingesetzt werden können. Als vorteilhaft haben sich hierfür beispielsweise poröse Materialien, wie Membranen, beispielsweise Nitrocellulosemembranen erwiesen. Es können jedoch auch faserige, saugfähige Matrixmaterialien wie Vliese, Gewebe oder Gewirke eingesetzt werden. Vliese sind besonders bevorzugt. Faserige Matrixmaterialien können Glas, Cellulose, Cellulosederivate, Polyester, Polyamid, aber auch Viskose, Zellwolle und Polyvinylalkohol enthalten. Vliese aus Fasern auf der Basis von Cellulose, Polymerisatfasern auf Basis von Polyester und / oder Polyamid und einem organischen Bindemittel, das OH- und / oder Estergruppen aufweist, wie sie aus EP-B-0 326 135 bekannt sind, sind beispielsweise erfindungsgemäß einsetzbar. Vliesmaterialien, enthaltend schmelzbare Copolyesterfasern neben Glasfasern, Polyesterfasern, Polyamidfasern, Cellulosefasern oder Cellulosederivatefasern, wie sie in der europäischen Patentanmeldung 0 571 941 beschrieben sind, können auch in dem Analysenelement eingesetzt werden. Papiere, wie beispielsweise Teebeutelpapier, sind ebenfalls gut einsetzbar.

Zur Verbesserung der Handhabung des Analysenelementes kann sich das saugfähige kapillaraktive Material oder können sich unterschiedliche saugfähige, kapillaraktive Materialien auf einem steifen Trägermaterial angeordnet befinden, welches seinerseits für Flüssigkeit nicht durchlässig ist, den Flüssigkeitsfluß im Matrixmaterial nicht negativ beeinflußt und sich in Bezug auf die im Analysenelement ablaufenden Reaktionen inert verhält. Bevorzugtes Trägermaterial kann beispielsweise Polyester-Folie sein, auf der das den Flüssigkeitstransport ermöglichende Matrixmaterial befestigt ist.

In dem Analysenelement können die einzelnen Zonen übereinander, nebeneinander oder teils übereinander und teils nebeneinander auf dem Trägermaterial angeordnet sein. Besonders bevorzugt ist ein Analysenelement, bei dem sich Probenauftrags- und Nachweiszone nebeneinander auf dem Trägermaterial angeordnet befinden. Nebeneinander bedeutet in diesem Zusammenhang, daß sich diese Zonen nebeneinander in direktem Kontakt miteinander befinden oder aber durch andere Zonen getrennt im wesentlichen in einer Ebene angeordnet sind.

Die Probenauftragszone ist der Bereich des Analysenelementes, auf den die Probe aufgegeben wird, in der bestimmt werden soll, ob ein bestimmter Analyt oder eine vom Analyt abgeleitete und diesen repräsentierende Substanz anwesend, gegebenenfalls in welcher Menge er bzw. sie anwesend ist.

Die Nachweiszone ist der Bereich des Analysenelementes, in dem die Bestimmung erfolgt, ob der untersuchte Analyt bzw. die vom Analyt abgeleitete und diesen repräsentierende Substanz in der auf das Analysenelement gegebenen Probe vorhanden war. Diese Bestimmung kann qualitativ, halb quantitativ oder quantitativ sein. Halb quantitativ bedeutet hierbei, daß für den Analyt bzw. die vom Analyt abgeleitete und diesen repräsentierende Substanz kein konkreter Konzentrationswert, sondern ein Konzentrationsbereich ermittelt wird, in dem sich die Analytkonzentration befindet.

In der Nachweiszone befindet sich Partner 1 eines spezifischen Bindungspaares 1 so immobilisiert, daß er in der Lage ist, mit Partner 2 des spezifischen Bindungspaares 1, der nicht der Analyt ist, eine Bindung einzugehen, wenn dieser ihn kontaktiert. Die Immobilisierung kann durch chemische Reaktion, das heißt, durch Ausbildung einer kovalenten Bindung, erzeugt worden sein. Sie kann aber auch durch adsorptive Kräfte erfolgen, welche alle Möglichkeiten außer der Ausbildung kovalenter Bindungen einschließt. Häufig wird für die Nachweiszone eine Nitrocellulosemembran verwendet, an die Proteine, aber auch Nukleinsäuren bei Imprägnieren fest binden, ohne jedoch eine kovalente Bindung einzugehen.

Erfindungsgemäß muß sich außer Partner 1 eines spezifischen Bindungspaares 1 auch ein markierter Partner 1 eines spezifischen Bindungspaares 2 in dem Analysenelement befinden. Dieser Partner darf nicht immobilisiert sein, sondern muß durch Flüssigkeit ablösbar imprägniert vorliegen, das heißt, dieser markierte Partner muß in Richtung der Nachweiszone durch Flüssigkeit transportabel sein. Vorteilhafterweise muß dieser markierte Partner durch möglichst wenig Flüssigkeit von dem Matrixmaterial, auf dem er imprägniert vorliegt, vollständig, das heißt, quantitativ, abgelöst werden können. Als Matrixmaterial haben sich hierfür Vliese als besonders geeignet erwiesen, wie sie beispielsweise in EP-B-0 326 135 beschrieben sind.

Spezifische Bindungspaare sind aus dem Stand der Technik bekannt und umfassen beispielsweise Paare wie Hapten und Antikörper, Antigen und Antikörper, Lectin und Zucker oder Saccharid, Avidin oder Streptavidin und Biotin sowie Nukleinsäure und Nukleinsäure, Ligand und Rezeptor. Ein Antigen kann hierbei jedes Molekül sein, gegen das man experimentell in der Lage ist, Antikörper zu erzeugen. Auch ein Antikörper oder eine bestimmte Stelle eines Antikörpers, die als Epitop bezeichnet wird, kann ein Antigen sein und von einem Antikörper spezifisch erkannt und gebunden werden. Unter Nukleinsäuren sollen alle möglichen Formen von Nukleinsäuren verstanden werden, die in der Lage sind, Bindungen über komplementäre Basen einzugehen. Speziell, aber nicht als abschließende Liste seien DNA, RNA, aber auch Nukleinsäureanaloga, wie Peptidnukleinsäuren (PNA, siehe beispielsweise in WO 92/20702), genannt. Ligand und Rezeptor bezeichnen ganz allgemein eine spezifische Bindungswechselwirkung zwischen zwei Partnern, wie beispielsweise zwischen Hormon und Hormonrezeptor.

In einer bevorzugten Ausführungsform ist Partner 1 eines spezifischen Bindungspaares 1 ein Antikörper, der ein Epitop auf einem anderen Antikörper, der gegen den Analyt gerichtet ist, erkennt. Das Epitop, gegen das der Antikörper gerichtet ist, entspricht dann Partner 2 des spezifischen Bindungspaares 1. Ganz besonders bevorzugt wird als Partner 1 des spezifischen Bindungspaares 1 jedoch Avidin oder Streptavidin verwendet, welches eine spezifische Bindung mit Biotin einzugehen in der Lage ist. Biotin bildet dann Partner 2 des spezifischen Bindungspaares 1.

In einer bevorzugten Ausführungsform ist Partner 1 des spezifischen Bindungspaares 2 ein Antikörper gegen Partner 2 des spezifischen Bindungspaares 2. Dieser Partner 2 des spezifischen Bindungspaares 2 ist erfindungsgemäß bevorzugt ein Hapten, vorteilhafterweise ein in der zu untersuchenden Probe nicht vorhandenes Hapten. Ganz besonders bevorzugt wird Digitoxigenin, Digitoxin, Digoxigenin oder Digoxin als Hapten eingesetzt.

Als Markierung des Partners 1 des spezifischen Bindungspaares 2 sind grundsätzlich alle Markierungen möglich, die für Immunoassays aus dem Stand der Technik bekannt sind. Dies sind insbesondere radioaktive Markierungen oder Enzymlabel wie beispielsweise Peroxidase, alkalische Phosphatase oder Galaktosidase oder Fluorophore. Besonders bevorzugt werden jedoch sogenannte Direktlabel eingesetzt, das heißt, Markierungen, die ohne weitere Handhabungsschritte mit dem Auge durch ihre Farbe erkennbar sind. Vorteilhafte Markierungen dieser Art sind beispielsweise in Wasser unlösliche Partikel wie Metall- oder Latexpartikel, aber auch Pigmente wie Silicat, Ruß oder Selen. Insbesondere Metallpartikel werden erfindungsgemäß bevorzugt als Markierung eingesetzt. Kolloidales Gold ist als Markierung besonders bevorzugt. Die Markierung kann an Partner 1 des spezifischen Bindungspaares 2 kovalent oder auch adsorptiv gebunden sein, wobei adsorptiv alle Möglichkeiten außer kovalenter Bindung einschließt. Im Falle farbiger Latexpartikel als Direktmarkierung liegt bevorzugt eine kovalente Bindung vor. Bei kolloidalen Metallen als Direktmarkierungen, insbesondere bei kolloidalem Gold werden vorzugsweise adsorptive Bindungen genutzt.

Die Herstellung von Antikörper-Gold-Konjugaten ist beispielsweise aus Roth, J. "The colloidal gold marker system for light and electron microscopic cytochemistry", in Bullock, G.R. and Petrusz, P. eds, "Techniques in Immunocytochemistry", Vol. 2., New York, Academic Press, 1983, S. 216-284 bekannt.

Der markierte Partner 1 des spezifischen Bindungspaares 2 kann sich an unterschiedlichen Stellen des Analysenelementes befinden. Dies ist abhängig beispielsweise von der beabsichtigten Reaktionsführung, von der Menge an zur Verfügung stehender Probe bzw. abhängig von der Analytkonzentration, wenn das Analysenelement zur Bestimmung flüssiger Proben bestimmt ist.

So kann sich der markierte Partner 1 des spezifischen Bindungspaares 2 in der Probenauftragszone befinden, er kann stromabwärts der Probenauftragszone zwischen Probenauftragszone und Nachweiszone angeordnet sein oder er kann sich auch stromaufwärts der Probenauftragszone befinden. Zumindest der letzte Fall setzt voraus, daß das Analysenelement außer der Probenauftragszone auch noch eine Elutionsmittelauftragszone besitzt. Eine solche Elutionsmittelauftragszone befindet sich dann entweder stromaufwärts des Bereiches, an dem sich der markierte Partner 1 des spezifischen Bindungspaares 2 befindet oder dieser Bereich, wo der markierte Partner 1 des spezifischen Bindungspaares 2 vorliegt, ist identisch mit der Elutionsmittelauftragszone. Insofern kann eine Elutionsmittelauftragszone stromaufwärts der Probenauftragszone oder an der Stelle der Probenauftragszone auf dem Analyseelement vorliegen. Eine solche Elutionsmittelauftragszone wird unabhängig davon, wo sich der markierte Partner 1 des spezifischen Bindungspaares 2 befindet, bevorzugt immer dann vorgesehen sein, wenn die zu untersuchende Probe nicht flüssig ist oder nicht so viel Flüssigkeit darstellt, daß sie für die Bestimmung des Analyten, das heißt, für den Transport des Analyten und der benötigten Reagenzien bis in die Nachweiszone, ausreicht.

Ein wie vorstehend beschriebener Aufbau eines Analyseelements ist universell für die Bestimmung jedes Analyt geeignet, der durch spezifische Paarbindung nachweisbar ist. Hierfür muß lediglich eine von Analyt abgeleitete und diesen repräsentierende Substanz, die Partner 2 eines spezifischen Bindungspaares 1 und Partner 2 eines spezifischen Bindungspaares 2 beinhaltet in einem Analysenelement mit markiertem Partner 1 des spezifischen Bindungspaares 2 in Kontakt gebracht werden, durch Flüssigkeitstransport in dem Analysenelement in Richtung der flußabwärts der Probenauftragszone gelegenen Nachweiszone bewegt, in der Nachweiszone an Partner 1 des spezifischen Bindungspaares 1 gebunden und anhand der Markierung des Partners 1 des spezifischen Bindungspaares 2 bestimmt werden. Für diese Bestimmung ist es besonders vorteilhaft, wenn bewegliche, nicht in der Nachweiszone immobilisierte Reaktionskomponenten durch Flüssigkeit aus der Nachweiszone entfernt werden. Wenn im Falle einer flüssigen Probe die Probenflüssigkeit zur Entfernung der beweglichen, nicht immobilisierten Reaktionskomponenten aus der Nachweiszone nicht ausreicht, kann zusätzliche Flüssigkeit auf das Analysenelement aufgegeben werden, wobei diese Aufgabe auf die Probenaufgabezone oder eine spezifische Elutionsmittelaufgabezone erfolgen kann.

Die den Analyt repräsentierende Substanz kann auf unterschiedliche Art und Weise erzeugt werden. Im Falle eines Antigens als Analyt ist es beispielsweise möglich, daß der Analyt mit zwei Antikörpern umgesetzt wird, die an den Analyt binden. Einer der beiden Antikörper trägt hierbei Partner 2 des spezifischen Bindungspaares 1, der andere Antikörper trägt Partner 2 des spezifischen Bindungspaares 2. Wenn der Analyt beispielsweise ein bestimmtes Epitop mehrfach enthält, ist es möglich, daß die beiden Antikörper identisch sind. Erfindungsgemäß ist es nicht unbedingt notwendig, daß die Mischung aus Analyt, Antikörper mit Partner 2 des spezifischen Bindungspaares 1 und Antikörper mit Partner 2 des spezifischen Bindungspaares 2 erst dann mit Partner 1 des spezifischen Bindungspaares 2 auf dem Analysenelement in Kontakt gebracht wird, wenn der Sandwich-Komplex zwischen den beiden Antikörpern und Analyt vollständig ausgebildet ist. Wichtig ist letztendlich, daß in der Nachweiszone im Zeitpunkt der Bestimmung des Analysenergebnisses ein Sandwich-Komplex an Partner 1 des spezifischen Bindungspaares 1 gebunden vorliegt. Diese Sandwich-Bildung kann bereits bei Aufgabe der Mischung aus Analyt und den beiden Sandwich bildenden Antikörpern auf das Analysenelement abgeschlossen sein, sie kann sich aber auch noch während des Flüssigkeitstransportes der Reagenzien zwischen Progenauftragszone und Nachweiszone vollziehen. Im äußersten Fall findet die Vervollständigung der Sandwich-Reaktion in der Nachweiszone statt. Der Begriff "von Analyt abgeleitete und diesen repräsentierende Substanz" umfaßt deshalb auch eine Mischung der eine solche Substanz erzeugenden Komponenten, sofern diese Komponenten zumindest in der Nachweiszone die von Analyt abgeleitete und diesen repräsentierende Substanz ergeben.

Im Falle der Bestimmung eines Antikörpers als Analyt können in Analogie zu der vorbeschriebenen Reaktion statt der beiden Antikörper Antigene oder das Antigenepitop repräsentierende Oligopeptide verwendet werden, wobei ein Teil der Antigenmoleküle Partner 2 des spezifischen Bindungspaares 1 trägt und der andere Teil der Antigenmoleküle Partner 2 des spezifischen Bindungspaares 2. Zur Bestimmung wird ein Doppelantigen-Sandwich-Komplex aus zu bestimmenden Antikörper und je einem Antigen mit Partner 2 des spezifischen Bindungspaares 1 und einem Antigen mit Partner 2 des spezifischen Bindungspaares 2 gebildet, für den das zuvor für die Antigenbestimmung ausgeführte analog gilt.

Der Aufbau des universell einsetzbaren Analysenelementes ist auch für die Bestimmung von Nukleinsäuren hervorragend geeignet. Hierzu muß die zu bestimmende Nukleinsäure oft vervielfältigt werden, um ausreichende Mengen für einen Nachweis zur Verfügung zu haben. Dies kann beispielsweise mittels der dem Fachmann bekannten Polymerase Chain Reaction (PCR) oder Ligase Chain Reaction (LCR) erfolgen. Bei einer Vervielfältigung mittels PCR, bei der ausgehend von einem als Primer eingesetzten Oligonukleotid Nukleotide zu einer zu der zu bestimmenden Nukleinsäure komplementären Nukleinsäure verknüpft und an den Primer angeknüpft werden, kann beispielsweise Partner 2 des spezifischen Bindungspaares 1 oder Partner 2 des spezifischen Bindungspaares 2 gebunden an ein solches Nukleotid oder einen Primer in die Kopie der Nukleinsäure eingebaut werden. Wird das so erhaltene Amplifikationsprodukt mit Nukleinsäure hybridisiert, die Partner 2 desjenigen Bindungspaares trägt, den die komplementäre vervielfältigte Nukleinsäure nicht aufweist, steht eine vom Analyt abgeleitete und diesen repräsentierende Substanz zur Verfügung, die auf dem Analysenelement an den in der Nachweiszone immobilisierten Partner 1 des spezifischen Bindungspaares 1 fixiert und mit dem markierten Partner des spezifischen Bindungspaares 2 bestimmbar gemacht worden ist.

Desweiteren ist es möglich bei ausreichenden Nukleinsäuremengen auf eine Vervielfältigung zu verzichten oder die Vervielfältigung der Nukleinsäure ohne Partner 2 der spezifischen Bindungspaare 1 oder 2 durchzuführen. Anschließend wird die den Analyt repräsentierende Substanz durch Hybridisierung zweier Sonden, die jeweils Partner 2 des spezifischen Bindungspaares 1 und Partner 2 des spezfisichen Bindungspaares 2 tragen, erzeugt.

Besonders bevorzugt wird für eine Vervielfältigung von zu bestimmender Nukleinsäure ein Partner 2 des spezifischen Bindungspaares 1 oder Partner 2 des spezifischen Bindungspaares 2 tragendes Nukleotid gemischt mit unmarkierten Nukleotiden verwendet und die vervielfältigte Partner 2 des spezifischen Bindungspaares 1 oder Partner 2 des spezifischen Bindungspaares 2 tragende Nukleinsäure mit einer Nukleinsäure hybridisiert, die Partner 2 desjenigen spezifischen Bindungspaares trägt, das nicht in der für die Vervielfältigung benutzten Nukleotidmischung vorhanden war. So wird ein Nukleinsäuredoppelstrang erhalten, in dem jeder Strang unterschiedliche Partner von spezifischen Bindungspaaren trägt. Statt eines Partner 2 des spezifischen Bindungspaares 1 oder Partner 2 des spezifischen Bindungspaares 2 tragende Nukleotids kann auch ein eben diesen Partner tragendes Oligonukleotid als Primer zusammen mit unmarkierten Nukleotiden verwendet werden. Als Partner 2 des spezifischen Bindungspaares 1 wird besonders bevorzugt Biotin und als Partner 2 des spezifischen Bindungspaares 2 ein Hapten, wie beispielsweise Fluorescein, Rhodamin, Digoxin oder ganz bevorzugt Digoxigenin verwendet. Als besonders vorteilhaft bei der Verwendung von Primern haben sich solche erwiesen, die biotinyliert sind.

Dadurch, daß biotinylierte Nukleotide und Primer kommerziell erhältlich sind oder Kits erhältlich sind, mit denen solche Substanzen wie Hapten, beispielsweise Fluorescein, Rhodamin, Digoxin oder besonders Digoxigenin tragende Nukleinsäuren oder Nukleinsäurefragmente herzustellen sind, ist es für den Fachmann sehr leicht möglich, insbesondere für Forschungszwecke, von zu bestimmenden Nukleinsäuren abgeleitete und diese repräsentierende Substanzen zu erhalten und mittels des Analysenelementes schnell und einfach nachzuweisen.

Für die Bestimmung von Analyten ist es natürlich auch möglich, einen Kit zur Verfügung zu stellen, der nicht nur das universell einsetzbare Analysenelement aufweist, sondern auch Partner 2 des spezifischen Bindungspaares 1 oder Partner 2 des spezifischen Bindungspaares 2 oder beide Partner. Diese Partner sind beispielsweise an ein Nukleotid, Oligonukleotid, eine Nukleinsäure, einen Antikörper, ein Hapten oder ein Antigen bzw. ein Epitop oder an ein Lectin oder an einen Rezeptor für einen Liganden konjugiert. Diese Substanzen haben die bereits früher erläuterte Bedeutung. So ist es möglich, dem Interessierten neben dem universell einsetzbaren Analysenelement Teile oder auch alle benötigten weiteren Reagenzien für die Bestimmung eines speziellen Analyts zur Verfügung zu stellen. Hierbei ist es im wesentlichen unerheblich, ob sich die Partner 2 des spezifischen Bindungspaares 1 und Partner 2 des spezifischen Bindungspaares 2 in getrennten Behältnissen oder zusammen in einem Behältnis befinden. Dies trifft insbesondere für solche Systeme zu, bei denen der zu bestimmende Analyt mittels zwei Antikörpern oder mittels Antigenen über einen Sandwich-Komplex nachgewiesen wird. Soll ein Kit zur Bestimmung einer Nukleinsäure zusammengestellt werden, ist es vorteilhaft, daß Partner 2 des spezifischen Bindungspaares 1 oder Partner 2 des spezifischen Bindungspaares 2 tragende Nukleotide oder Primer getrennt aufbewahrt werden von einer der zu bestimmenden Nukleinsäure komplementären Nukleinsäure die Partner 2 desjenigen spezifischen Bindungspaares trägt, der von dem Partner, der mit dem Nukleotid bzw. dem Primer konjugiert ist, verschieden ist.

Das beschriebene universell einsetzbare Analysenelement bietet vor allem Vorteile bei Analytbestimmungen in Forschung und Entwicklung, wo der Analyt sehr unterschiedlich sein kann. Besonders vorteilhaft ist das Analysenelement insbesondere für die Bestimmung von Nukleinsäuren einsetzbar. Nukleotide und Oligonukleotide, die mit Partnern 2 eines spezifischen Bindungspaares 1 bzw. Partner 2 eines spezifischen Bindungspaares 2 konjugiert sind, sind in einer breiten Vielfalt kommerziell erhältlich. Das gleiche trifft auch für Partner 2 eines spezifischen Bindungspaares 1 oder Partner 2 eines spezifischen Bindungspaares 2 tragende Nukleinsäuresonden zu, die leicht mit kommerziell erhältlichen Kits hergestellt werden können. Insbesondere Biotin und / oder Digoxin bzw. Digoxigenin tragende Nukleinsäuresonden sind so leicht erhältlich.

Der universell einsetzbare Aufbau des Analysenelementes ist auch deshalb vorteilhaft, weil üblicherweise bei Immunoassays mit markierten Bindungspartnern des Analyts der markierte Bindungspartner eine kritische Komponente darstellt. Bisher war es üblich, je nach Analyt einen entsprechenden markierten Bindungspartner herzustellen, für den dann auf dem Analysenelement optimale Bedingungen für Reaktion und Lagerung geschaffen werden mußten. Dies erforderte in der Vergangenheit einen hohen Aufwand. Mit dem Analysenelement wird jetzt ein Element zur Verfügung gestellt, das universell anwendbar ist. Die spezifischen Reagenzien können kurzfristig und zu niedrigen Kosten als Flüssigreagenzien hergestellt werden. Optimierungsarbeit insbesondere in Bezug auf die Lagerfähigkeit solcher Reagenzien auf Analysenelementen entfällt.

Darüberhinaus ist es aber auch möglich, falls dies gewünscht wird, das Analysenelement nicht nur als universelles Analysenelement zusammen mit den spezifischen Reagenzien als Flüssigreagenzien zu verwenden, sondern, ausgehend von dem Analysenelement ein Analyseelement zu erzeugen, das die für den spezifischen Nachweis eines Analyts erforderliche Gesamtheit aller Reagenzien enthält. Für einen Antigennachweis mittels Sandwichkomplexbildung können so beispielsweise die erforderlichen Antikörper, sofern der eine mit Partner 2 eines spezifischen Bindungspaares 1, der andere mit Partner 2 eines spezifischen Bindungspaares 2 konjugiert ist, auf einem Analysenelement integriert vorliegen. Gleiches gilt auch für ein Analysenelement, das für den Nachweis eines Antikörpers mittels Sandwichkomplexbildung bestimmt ist. Hier liegt ein Teil des erforderlichen Antigens konjugiert mit Partner 2 eines spezifischen Bindungspaares 1 und der andere Teil des Antigens konjugiert mit Partner 2 eines spezifischen Bindungspaares 2 auf dem Analysenelement integriert vor. Solche Konjugate können in einer gemeinsamen Zone oder in benachbarten, übereinander oder nebeneinander angeordneten Zonen des Analysenelements angeordnet sein. Hierbei können dann beide Konjugate in der Probenauftragszone oder eines der Konjugate in der Probenauftragszone und das andere in einer Zone zwischen Probenauftrags- und Nachweiszone oder beide Konjugate getrennt oder zusammen in einer Zone zwischen Probenauftragsund Nachweiszone untergebracht sein. Im Falle einer stromaufwärts der Probenauftragszone angeordneten Elutionsmittelzone gibt es außer den zuvor aufgeführten Möglichkeiten auch noch die, daß die die Partner 2 der spezifischen Bindungspaare 1 und 2 tragenden Antigene oder Antikörper zwischen Elutionsmittel- und Probenauftragszone getrennt oder zusammen angeordnet sind. Solche, alle erforderlichen Reagenzien zur Bestimmung eines Analyts enthaltenden Analysenelemente beinhalten die Vorteile des einfachen und universellen Aufbaus und sind für den Benutzer enorm einfach zu handhaben, da nur eine Probe aufgegeben werden muß, ansonsten aber keine Handhabungsschritte mehr erforderlich sind, bevor in der Nachweiszone das Ergebnis abgelesen wird.

Ein Analysenelement ist auch zur Bestimmung mindestens eines von mehreren Analyten einsetzbar. Beispielsweise gilt es heute bei der Untersuchung von Proben auf eine HIV-Infektion festzustellen, ob einer von mehreren möglichen Antikörpertypen, nämlich Antikörper gegen HIV 1, gegen HIV 2 oder gegen HIV 1 Subtyp O vorhanden ist. Die Anwesenheit von Antikörpern gegen einen Typus reichen aus, eine Probe als positiv zu bewerten. Gegen welchen Typus die gefundenen Antikörper genau gerichtet sind, ist zumindest bei Screeningverfahren, erst von nachgeordneter Bedeutung. Ein Analysenelement, das für eine solche Bestimmung geeignet ist, enthält jeweils ein Paar von Antigenkonjugaten gegen jeden Antikörper, auf den hin eine Probe untersucht werden soll. Jedes Paar enthält Antigen konjuguert mit Partner 2 des spezifischen Bindungspaares 1 und Antigen konjugiert mit Partner 2 des spezifischen Bindungspaares 2, wobei das Antigen spezifisch an einen bestimmten Antikörpertypus bindet. Die Antigenkonjugate können jeweils getrennt vorliegen. Es ist aber auch möglich, daß alle Antigenkonjugate gemischt sind und zusammen in einer Zone untergebracht sind. Für die Lokalisierung solcher Konjugate in einem Analysenelement gelten die zuvor ganz allgemein für Konjugate von Antigenen oder Antikörpern mit Partnern 2 von spezifischen Bindungspaaren 1 und 2 gemachten Ausführungen.

Ein analoges Analysenelement für die Bestimmung von Influenza kann das Vorliegen von Influenzaviren A und / oder Influenzaviren B nachweisen. Hierzu werden Konjugate von Antikörpern gegen Influenza A-Viren mit Partnern 2 von spezifischen Bindungspaaren 1 und 2 sowie Konjugate von Antikörpern gegen Influenza-Viren B mit Partnern 2 der spezifischen Bindungspaare 1 und 2 eingesetzt. Bei Vorliegen mindestens eines der beiden Virentypen zeigt das Analysenelement in der Nachweiszone einen positiven Befund an.

Darüberhinaus ist es möglich, daß ein Analysenelement auch noch weitere funktionelle Zonen enthält. Beispielsweise hat es sich als vorteilhaft erwiesen für die Untersuchung von Vollblut in dem Analysenelement eine Zone vorzusehen, in der Plasma bzw. Serum als klare Flüssigkeit von Vollblut abgetrennt wird und Blutzellen zurückgehalten werden. Nur die klare Flüssigkeit wird dann bis in die Nachweiszone transportiert. Für die Trennung von Plasma bzw. Serum aus Vollblut sind beispielsweise Glasfaservliese geeignet, wie sie in EP-A-0 045 476 beschrieben sind. Ein solches für die Abtrennung von Plasma bzw. Serum aus Vollblut geeignetes Medium kann beispielsweise in der Probenauftragszone oder zwischen Probenauftragszone und Nachweiszone angeordnet sein.

Ausgehend von dem universell einsetzbaren Aufbau des Analysenelementes ist die Entwicklung von Analysenelementen, die alle Reagenzien zum Nachweis eines oder mehrerer Analyte tragen, im Hinblick auf den bisherigen Entwicklungsaufwand wesentlich vereinfacht und Entwicklungszeiten können so verkürzt werden.

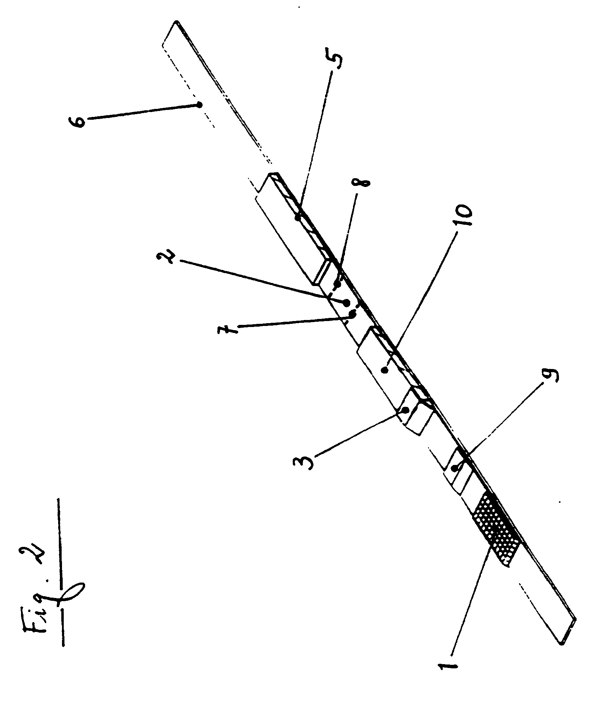

Zwei besonders bevorzugte Analysenelemente sind in Fig. 1 und 2 abgebildet.

Fig. 1 zeigt ein universell einsetzbares Analysenelement, das auf einer Trägerfolie (6),
eine Elutionsmittelaufgabezone (4),
eine Zone mit markiertem Partner 1 des spezifischen Bindungspaares 2 (3),
eine Probenaufgabezone (1),
eine Nachweiszone (2) mit einem farblosen Nachweisstrich (7), enthaltend immobilisierten Partner 1 des spezifischen Bindungspaares 1 und einem farblosen Kontrollstrich (8), der einen Antikörper gegen Partner 1 des spezifischen Bindungspaares 2 enthält,
sowie eine Flüssigkeitssammelzone (5) besitzt. Die Zonen sind nebeneinander, im wesentlichen in einer Ebene, auf der Trägerfolie (6) angeordnet, wobei "nebeneinander" hier jeweils eine leichte Überlappung der in Flüssigkeitstransportrichtung vorhergehenden mit der nachfolgenden Zone mit einschließt, damit der Flüssigkeitsübergang von einer Zone in die andere gewährleistet ist.

Das Analyseelement, das in Fig. 2 dargestellt ist, ist ein vollintegriertes Analysenelement, das heißt, es besitzt alle für die Durchführung einer Analytbestimmung notwendigen Reagenzien. Es ist außerdem für die Untersuchung von Vollblut geeignet. Auf einer Trägerfolie (6) sind
eine Probenaufgabezone (1),
eine Zone mit den Partnern 2 der spezifischen Bindungspaare 1 und 2 (9),
eine Zone mit markiertem Partner 1 des spezifischen Bindungspaares 2 (3),
eine Plasma- bzw. Serumabtrennzone (10),
eine Nachweiszone (2) mit einem farblosen Nachweisstrich (7), enthaltend immobilisierten Partner 1 des spezifischen Bindungspaares 1, und einem farblosen Kontrollstrich (8), der einen Antikörper gegen Partner 1 des spezifischen Bindungspaares 2 enthält
sowie eine Flüssigkeitssammelzone (5) nebeneinander angeordnet.

Die Erfindung wird durch die nachfolgenden Beispiele näher erläutert.

### Beispiel 1

### Bestimmung von Nukleinsäure

### a) Analysenelement

Es wurde ein Teststreifen gemäß Figur 1 hergestellt. Auf einer 5 mm breiten und 10 cm langen Trägerfolie (6) aus Polyester (Melinex^{®}, 350 µm dick von Imperial Chemistry Industries, Großbritannien) wurden mit Schmelzkleber (Dynapol^{®} S 1358 von Hüls AG, Deutschland)
- ein 1,5 mm dickes und 1,5 cm langes Vlies aus 100 Teilen Glasfasern (Durchmesser 0,49 bis 0,58 µm, Länge 1000 µm) und 5 Teilen Polyvinylalkoholfasern (Kuralon^{®}VPB 105-2 von Kuraray) mit einem Flächengewicht von 180 g / m² als Flüssigkeitsammelzone (5),
- eine 1,5 cm lange Cellulosenitrat-Membran (Typ CN 11301 von Sartorius, Deutschland) als Nachweiszone (2),
- ein 8 mm langes Vlies enthaltend 80 Teile Polyesterfasern, 20 Teile Zellwolle und 20 Teile Polyvinylalkohol mit einer Dicke von 0,32 mm und einem Flächengewicht von 80 g / m², dessen Herstellung in Beispiel 1 der europäischen Patentschrift 0 326 135 beschrieben ist als Probenaufgabezone (1),
- ein 8 mm langes Vlies aus 80 Teilen Polyesterfasern, 20 Teilen Zellwolle und 20 Teilen Polyvinylalkoholfasern, einer Dicke von 0,32 mm und einem Flächengewicht von 80 g / m²,
- dessen Herstellung in der europäischen Patentschrift 0 326 135, Beispiel 1 beschrieben ist, enthaltend Goldkonjugat als Zone mit markiertem Partner des spezifischen Bindungspaares 2 (3) und
- ein 30 mm langes Vlies (Typ Binzer TI 05 von Binzer, Deutschland) als Elutionsmittelaufgabezone (4) leicht überlappend nebeneinander befestigt.

### Zur Nachweiszone (2):

Auf die zuvor beschriebene Zellulosenitratmembran wurden durch Strichdosierung eine wässrige Streptavidinlösung (7 mg / ml) aufgebracht. Hierzu wurde die Dosierung so gewählt, daß ein Strich mit einer Breite von ca. 0,5 mm entsteht. Dieser Strich (7) dient zum Nachweis des zu bestimmenden Analyts. Die Membran wurde anschließend luftgetrocknet.

In einem Abstand von etwa 4 mm von dem Streptavidinstrich wurde durch Strichdosierung eine wässrige Lösung eines polyklonalen Antikörpers aus Kaninchen-IgG gegen Maus IgG (Bezugsquelle: DAKO Diagnostica GmbH, Hamburg, Deutschland) (0,5 mg / ml) aufgebracht. Auch hier wurde die Dosierung so gewählt, daß ein Strich mit einer Breite von ca. 0,5 mm entsteht. Dieser Strich (8) dient zur Funktionskontrolle des Teststreifens. Die Membran wurde anschließend luftgetrocknet.

### Zum Goldkonjugatvlies (3):

- Goldsol mit einem mittleren Partikeldurchmesser von ca. 40 nm wurde nach der Methode von Frens (Frens, G., "Preparation of gold dispersions of varying particle size: controlled nucleation for the regulation of the particle size in monodisperse gold suspensions" in Nature: Physical Science 241 (1973), 20-22) durch Reduktion einer 0,01 Gewichtsprozentigen Tetrachlorgoldsäurelösung unter Kochen mittels Trinatriumcitrat hergestellt.
- Die Antikörper-Goldkonjugat-Herstellung erfolgte in Anlehnung an die Methode von Roth, J. "The colloidal gold marker system for light and electron microscopic cytochemistry" in Bullock, G.R. and Petrusz, P., eds., "Techniques in Immunocytochemistry", Vol. 2, New York, Academic Press, 1983, 216-284.

Nach dem Abkühlen der wie zuvor beschriebenen Goldsollösung auf Raumtemperatur wurde der pH-Wert des Goldsols mit 0,2 M K₂CO₃ so eingestellt, daß er um 0,5 bis 1,0 pH-Einheiten über dem isoelektrischen Punkt des Antikörpers lag. Die optische Dichte (OD) des Goldsols (Extinktion bei 525 nm und 1 cm Lichtweg) betrug typischerweise 1,0. Zum Goldsol wurde eine dialysierte Lösung eines monoklonalen IgG-Antikörpers gegen Digoxygenin (MAK <Digoxygenin> IgG) (Bezugsquelle Boehringer Mannheim GmbH, Deutschland) zugegeben. Die Menge an Antikörperlösung wurde so gewählt, daß dessen Konzentration in der Goldsollösung typischerweise bei 2 µg / ml lag. Nach 30 Minuten Rühren bei Raumtemperatur wurde das Goldkonjugat durch Zugabe einer hochkonzentrierten Rinderserumalbumin-Lösung (Endkonzentration in der Konjugatlösung: 1mg / ml) abgesättigt.

Das Goldkonjugat wurde durch Ultrafiltration gegen einen 20 mM Tris-Puffer pH 8,0 auf eine optische Dichte von typischerweise 20 aufkonzentriert. Die Konjugatlösung wurde abschließend auf 100 µM Brij^{®} und 0,05 Gew.-% NaN₃ aufgestockt.

- Das so hergestellte Goldkonjugat (optische Dichte, OD = 20) wurde mit einem Tränkpuffer in einem Volumenverhältnis 1:1 auf eine optische Dichte, OD = 10 eingestellt. Der Tränkpuffer enthielt die nachfolgenden Komponenten:
1 Gew.-% Sacharose
200 mM HEPES
100 mM NaCl
140 mM Harnstoff
6 mM N-Acetylcystein
2 mM EDTA
0,1 Gew.-% Tween^{®}20.

Das Polyester-Zellwoll-Polyvinylalkohol-Mischvlies wurde mit einer konstanten Geschwindigkeit zunächst durch eine die Tränklösung enthaltende Wanne gezogen, anschließend durch zwei in einem Abstand von 250 µm angeordnete Edelstahlwalzen abgequetscht und anschließend mittels eines Umluftrockners getrocknet. Unter den beschriebenen Bedinungen beträgt die Tränkaufnahme des Vlieses typischerweise etwa 270 ml / m².

### b) Bestimmung eines Amplifikationsproduktes von Chlamydia trachomatis

Ein Fragment des kryptischen Plasmides (7,5 Kb) von Chlamydia trachomatis mit 143 Basenpaaren wurde amplifiziert. Hierzu wurden die Primer des Chlamydia trachomatis Primer and Capture Probe Set (Boehringer Mannheim, Deutschland) verwendet (Primer 1: 20-mer, Position 274-295; Primer 2: 24-mer, Position 393-416 rev.).
- Markierung erfolgte mit DIG-11-dUTP (DIG steht für Digoxygenin) und einer 5'-biotin-markierten Fangsonde (Boehringer Mannheim GmbH, Deutschland)
- Der PCR-Mastermix enthielt 2,5 U Taq-Polymerase, 10 µl 10-fachen PCR-Puffer einschließlich 25 mmolar Magnesiumchlorid, 0,2 µ molar beide Primer, 0,1 mmolar jedes Desoxynucleotid und 0,02 mmolar DIG-11-dUTP. Hieraus ergibt sich eine Markierungsstöchiometrie von 1:5 DIG-11-dUTP zu Desoxynnukleotide. Der Mastermix wurde durch Hinzupipettieren von 10 µl einer Lösung, die ca. 100 Kopien / µl des kryptischen Plasmidfragmentes enthielt und destilliertem Wasser auf 100 µl aufgefüllt. Der Mastermix wurde 10 min. bei 94°C geschmolzen und durchlief 35 mal einen Zyklus, bei dem er jeweils 40 Sekunden bei 94°C gehalten wurde, 30 Sekunden bei 52°C und 45 Sekunden bei 72° C. Das PCR-Produkt wurde in einem 2,5% Agarose-Gel kontrolliert, welches mit Ethidiumbromid gefärbt wurde.
- Zur Hybridisierung wurde 1 µl einer 5'-biotinmarkierten Fangsonde (Position 354 - 374) in einer Konzentration von 30 µmolar zu 50 µl des Amplifikationsproduktes gegeben. Die markierte Fangsonde lag so in einer Konzentration von 0,6 µ molar vor. Die Probe wurde danach für 5 Minuten bei 95° C aufgeschmolzen und anschließend für 15 Minuten bei 37° C hybridisiert.
- Zur Bestimmung der Nukleinsäure auf dem zuvor beschriebenen Teststreifen gemäß Fig. 1 wurden 5 µl des Hybridisierungsproduktes auf die Probenaufgabezone (1) gegeben. Danach wurde der Teststreifen mit der Elutionsmittelaufgabezone (4) für 5 Sekunden in einen Chromatographiepuffer getaucht, wobei darauf geachtet wurde, daß die Zone (3) mit dem Goldkonjugat nicht in die Flüssigkeit getaucht wurde. Der Chromatographiepuffer hatte die folgende Zusammensetzung: 0,9 Gew.-% Kochsalz, 50 mM Kaliumphosphat, 0,09 Gew.-% Natriumazid, 2 Gew.-% Rinderplasmaalbumin und 0,25 Gew.-% Tween^{®} 20.

Nach 10 Minuten war der Chromatographiepuffer aus der Elutionsmittelaufgabezone (4) bis in die Flüssigkeitssammelzone (5) gewandert. In der Nachweiszone (2) waren 2 rote Striche deutlich sichtbar, wobei der rote Nachweisstrich (enthaltend Streptavidin) einen positiven Befund signalisiert und der rote Kontrollstrich (enthaltend PAK <MAUS Fcγ〉) die ordnungsgenmäße Funktion des Analysenelementes.

### Beispiel 2

### Nachweis von Influenza A/B-Viren

### a) Analysenelement

Es wurde ein Teststreifen gemäß Fig. 1 hergestellt. Sämtliche Bestandteile des Analysenelements stimmten mit dem in Beispiel 1 beschriebenen Analysenelement zur Bestimmung von Nukleinsäure überein.

### b) Influenza-spezifische Immunoreagenzien

Antikörper zum Nachweis des Nukleoproteins von Influenza A- und Influenza B-Viren wurden erhalten von Fitzgerald Industries Int., Concord, Massachusetts, USA.
- Zur Herstellung eines biotinmarkierten monoklonalen Antikörpers aus Maus-IgG gegen das Nukleoprotein von Influenza A wurde zu einer Lösung von 20 mg / ml Antikörper in 0,1 M Kaliumphosphat pH 8,5 ein Succinimidesterderivat des Biotins in einem 6-fachen molaren Überschuß zugegeben. Der Ansatz wurde für 90 Minuten bei 25°C unter Rühren inkubiert. Die Reaktion wurde durch Aufstockung der Lösung mit Lysin auf eine Endkonzentration von 10 mM abgestoppt. Das überschüssige Biotinylierungsreagenz wurde durch Dialyse entfernt und die Lösung eingefroren.
- Für die Herstellung eines biotinylierten monoklonalen Antikörpers gegen das Nukleoprotein von Influenza B wurde analog vorgegangen.
- Zur Herstellung eines digoxigenylierten monoklonalen Antikörpers gegen das Nukleoprotein von Influenza A wurde zu einer Lösung von 10 mg / ml des monoklonalen Antikörpers in 0,1 M Kaliumphosphat ein in Dimethylsulfoxid (DMSO) gelöstes Succinimidester-Derivat des Digoxigenins in einem 4-fachen molaren Überschuß so zugegeben, daß die Endkonzentration an DMSO in der Lösung 5 Vol.% betrug. Der Ansatz wurde für 60 Minuten bei 25° C unter Rühren inkubiert. Die Reaktion wurde danach durch Zugabe einer 1 molaren wässrigen Lysinlösung abgestoppt, so daß die Endkonzentration an Lysin 10 mM war. Das überschüssige Digoxigenylierungsreagenz wurde mittels Dialyse gegen einen 20 mM Kaliumphosphatpuffer pH 8,0 entfernt und die Lösung bis zur Benutzung eingefroren.
- Die Herstellung eines digoxigenylierten monoklonalen Antikörpers gegen das Nukleoprotein von Influenza B erfolgte analog zu der vorbeschriebenen Digoxigenylierung des monoklonalen Antikörpers gegen das Nukleoprotein von Influenza A.

Die verwendeten Influenza A- und Influenza B-Antikörper sind typspezifisch, das heißt, der Influenza A-Antikörper erkennt lediglich das Nukleoprotein aus Influenza A-Viren, während der monoklonale Influenza B-Antikörper lediglich das Nukleoprotein aus Influenza B-Viren erkennt. Die Antikörper sind jedoch nicht Subtyp-spezifisch, das heißt, der monoklonale Antikörper gegen Influenza A erkennt alle Influenza A-Subtypen.

### c) Bestimmung von Influenza A- und / oder Influenza B-Viren

Als Probenmaterial zum Beleg der Sensitivität des erfindungsgemäßen Analysenelements wurden verdünnte Viruskulturüberstände verwendet. Die Viruskultivierung erfolgte auf MDCK-Zellen, einem permanenten Hundenierenzellstamm. Die Bebrühtung erfolgte im üblichen Kulturmedium für etwa 7 Tage bei 33° C. Als Vertreter für Influenza A wurde der Subtpy H3N2 (Stamm Beijing 32/92), als Vertreter für Influenza B der Stamm B/Harbin7/94 kultiviert. Die Kulturüberstände wurden mit Kulturmedium in Zweierschritten verdünnt.

Von jeder unterschiedlichen Verdünnung wurden 65 µl Kulturüberstand, 15 µl Lysepuffer (6% Zwittergent^{®} 3-10 in Rinderserumalbumin enthaltender physiologischer Kochsalzlösung) und jeweils 5 µl einer Lösung von biotinyliertem monoklonalen Antikörper gegen Influenza A und digoxigenylierten monoklonalen Antikörper gegen Influenza A zum Nachweis von Influenza A bzw. je 5 µl einer Lösung von biotinyliertem monoklonalen Antikörper gegen Influenza B und digoxigenyliertem monoklonalen Antikörper gegen Influenza B in ein Eppendorf-Gefäß pipettiert (die Konzentration der Antikörperkonjugatstammlösungen betrug jeweils 20 µg / ml). Die lysierte Probe wurde kurz durch Schütteln homogenisiert und anschließend 80 µl auf das Goldkonjugatvlies (3) des Teststreifens gemäß Fig. 1 pipettiert. Anschließend wurden die Teststreifen für ca. 5 Sekunden mit der Elutionsmittelaufgabezone (4) in den Chromatographiepuffer (0,9 Gew.-% Kochsalz, 50 mM Kaliumphosphat, 0,09 Gew.-% Natriumazid, 2 Gew.-% Rinderplasmaalbumin und 0,25 Gew.-% Tween^{®} 20) getaucht. Das Testergebnis wurde nach 10 Minuten in der Nachweiszone (2) abgelesen.

Beim Influenza A-Kulturüberstand konnte bis zu einer Kulturverdünnung von 1:64 ein roter Nachweisstrich zum Zeichen eines positiven Befundes erkannt werden. Beim Influenza B-Kulturüberstand wurden die Verdünnungen bis zur Stufe 1:128 positiv erkannt. Der Kontrollstrich zeigte durch seine rote Farbe in allen Fällen die ordnungsgemäße Funktion des Teststreifens an.

### Beispiel 3

### Nachweis von HIV-Antikörpern

### a) Analysenelement

Es wurde ein Teststreifen gemäß Figur 2 hergestellt. Auf einer 4 mm breiten und 10 cm langen Trägerfolie (6) aus Polyester (Melinex^{®}, 350 µm dick von Imperial Chemistry Industries, Großbritannien) wurden mit Schmelzkleber (Dynapol^{®} S 1358 von Hüls AG, Deutschland)
- ein 0,9 mm dickes und 1,4 cm langes Vlies aus 100 Teilen Glasfasern (Durchmesser 0,49 bis 0,58 µm, Länge 1000 µm) und 5 Teilen Polyvinylalkoholfasern (Kuralon^{®}VPB 105-2 von Kuraray) mit einem Flächengewicht von 100 g / m² als Flüssigkeitsammelzone (5),
- eine 1,5 cm lange Cellulosenitrat-Membran (Typ CN 11301 von Sartorius, Deutschland) als Nachweiszone (2),
- ein 1,2 cm langes Vlies aus 100 Teilen Glasfasern (Durchmesser 0,49 bis 0,58 µm, Länge 100 µm) und 5 Teilen Polyvinylalkoholfasern ((Kuralon^{®} VPB 105-2 von Kuraray) mit einem Flächengewicht von 100 g / m², das mit Brij^{®} (1 Gew.-%ig) imprägniert ist als Plasma- bzw. Serumtrennzone( 10),
- ein 12 mm langes Vlies aus 80 Teilen Polyesterfasern, 20 Teilen Zellwolle und 20 Teilen Polyvinylalkoholfasern, einer Dicke von 0,32 mm und einem Flächengewicht von 80 g / m², dessen Herstellung in der europäischen Patentschrift 0 326 135, Beispiel 1 beschrieben ist, enthaltend Goldkonjugat als Zone mit markiertem Partner des spezifischen Bindungspaares 2 (3),
- ein 12 mm langes Vlies aus 80 Teilen Polyesterfasern, 20 Teilen Zellwolle und 20 Teilen Poylvinylalkoholfasern, einer Dicke von 0,32 mm und einem Flächengewicht von 80 g / m², dessen Herstellung in der europäischen Patentschrift 0 326 135, Beispiel 1, beschrieben ist, enthaltend digoxigenylierte und biotinylierte HIV-Antigene als Zone mit den Partnern 2 der spezifischen Bindungspaare 1 und 2 (9)
   und
- ein 8 mm langes Polyestergewebe (PE 280 HC von Seidengaze Thal, Schweiz) mit Netzmittel imprägniert als Probenaufgabezone (1) leicht überlappend nebeneinander befestigt.

### Zur Nachweiszone (2):

Auf die zuvor beschriebene Zellulosenitratmembran wurden durch Strichdosierung eine wässrige Streptavidinlösung (4 mg / ml) aufgebracht. Hierzu wurde die Dosierung so gewählt, daß ein Strich mit einer Breite von ca. 0,4 mm entsteht. Dieser Strich dient zum Nachweis von HIV-Antikörpern. Die Membran wurde anschließend luftgetrocknet.

In einem Abstand von etwa 4 mm von dem Streptavidinstrich wurde durch Strichdosierung eine wässrige Lösung eines polyklonalen Antikörpers aus Kaninchen-IgG gegen Maus-IgG (Bezugsquelle: DAKO Diagnostica GmbH, Hamburg, Deutschland) (0,5 mg / ml) aufgebracht. Auch hier wurde die Dosierung so gewählt, daß ein Strich mit einer Breite von ca. 0,4 mm entsteht. Dieser Strich (8) dient zur Funktionskontrolle des Teststreifens. Die Membran wurde anschließend luftgetrocknet.

### Zum Goldkonjugatvlies (3):

- Goldsol mit einem mittleren Partikeldurchmesser von ca. 40 nm wurde, wie in Beispiel 1a beschrieben, hergestellt.
- Auch die Antikörper-Goldkonjugat-Herstellung erfolgte wie in Beispiel 1 a beschrieben.

Das so hergestellte Goldkonjugat (optische Dichte, OD = 20) wurde mit einem Tränkpuffer auf eine optische Dichte, OD = 3 (Extinktion bis 525 nm und 1 cm Lichtweg) eingestellt. Der Tränkpuffer enthielt die nachfolgenden Komponenten:
100 mM HEPES, pH 7,5
50 mM NaCl
0,5 Gew.-% Saccharose
70 mM Harnstoff
3 mM N-Acetylcystein
1 mM EDTA und
0,1 Gew.-% Tween^{®} 20

Das Polyester-Zellwoll-Polyvinylalkohol-Mischvlies wurde mit einer konstanten Geschwindigkeit zunächst durch eine die Tränklösung enthaltende Wanne gezogen, anschließend durch zwei in einem Abstand von 250 µm angeordnete Edelstahlwalzen abgequetscht und anschließend mittels eines Umluftrockners getrocknet. Unter den beschriebenen Bedinungen beträgt die Tränkaufnahme des Vlieses typischerweise etwa 270 ml / m².

### Zum Vlies (9) enthaltend digoxigenylierte und biotinylierte HIV-Antigene:

Die Herstellung digoxigenylierter Peptide bzw. biotinylierter Peptide aus dem Bereich gp 41 von HIV I sind in der internationalen Patentanmeldung WO 9603423 in Beispiel 1 beschrieben. Die jeweiligen Peptide kamen jeweils paarweise als Digoxigenin- und Biotinderivate zum Einsatz. Ihre Ansatzkonzentrationen in der Tränklösung lagen zwischen 0,7 · 10⁻⁷ Mol / l und 3 · 10⁻⁷ Mol/l. Die Tränklösung enthielt darüber hinaus
100 mM MES-Puffer, pH 6,0
50 mM NaCl
2 Gew.-% Saccharose
1 Gew.-% Rinderserumalbumin
3 mM N-Acetylcystein
0,06 Gew.-% Tween^{®} 20
1 mM EDTA

Das Polyester-Zellwoll-Polyvinylalkohol-Mischvlies wurde in dieser Tränklösung imprägniert und anschließend mittels eines Umlufttrockners getrocknet. Die Trankaufnahme beträgt typischerweise etwa 270 ml / m².

### b) Bestimmung einer HIV-Infektion

Auf die Probenaufgabezone (1) des zuvor beschriebenen Teststreifens gemäß Fig. 2 wurden etwa 60 µl Probenvolumen (Plasma bzw. Serum) aufgegeben. Nach 15 minütiger Wartezeit wurde die Nachweiszone (2) visuell ausgewertet. Ein rotvioletter Strich in der Position der Kontrollinie (8) bedeutet nichtreaktive Probe (keine HIV-Infektion nachweisbar). Zwei rotviolette Striche, einer in der Position der Kontrollinie (8) und einer in der Position der Nachweislinie (7) bedeuten reaktive Probe (HIV-Infektion nachweisbar).

## Patentansprüche

1. Verfahren zur Bestimmung eines Analyts mittels spezifischer Bindungspaare unter Verwendung eines Analyseelements enthaltend in oder auf Material, das einen Flüssigkeitstransport zwischen Zonen ermöglicht, eine Probenauftragszone und eine hierzu flussabwärts gelegene Nachweiszone, wobei die Nachweiszone Partner 1 eines spezifischen Bindungspaares 1 so immobilisiert enthält, dass er in der Lage ist, mit Partner 2 des spezifischen Bindungspaares 1, der nicht Analyt ist, eine Bindung einzugehen, wenn er ihn kontaktiert, und flussaufwärts der Nachweiszone ein markierter Partner 1 eines spezifischen Bindungspaares 2 auf einem Material durch Flüssigkeit ablösbar imprägniert vorliegt und in der Lage ist, mit Partner 2 des spezifischen Bindungspaares 2, der nicht der Analyt ist, eine Bindung einzugehen, wenn dieser ihn kontaktiert,
**dadurch gekennzeichnet, dass**
eine vom Analyt abgeleitete und diesen repräsentierende Substanz, die Partner 2 des spezifischen Bindungspaares 1 und Partner 2 des spezifischen Bindungspaares 2 beinhaltet, in dem Analyseelement mit dem markierten Partner des spezifischen Bindungspaares 2 in Kontakt gebracht, durch Flüssigkeitstransport in dem Analysenelement in Richtung der flussabwärts der Probenauftragszone gelegenen Nachweiszone bewegt, in der Nachweiszone an Partner 1 des spezifischen Bindungspaares 1 gebunden und anhand der Markierung des Partners 1 des spezifischen Bindungspaares 2 bestimmt wird.

2. Verfahren gemäß Anspruch 1, **dadurch gekennzeichnet, dass** zusammengehörige Partner von spezifischen Bindungspaaren ausgewählt sind aus der Gruppe Hapten und Antikörper, Antigen und Antikörper, Lectin und Zucker / Saccharid, Ligand und Rezeptor, Avidin / Streptavidin und Biotin, Nukleinsäure und Nukleinsäure.

3. Verfahren gemäß Anspruch 1, **dadurch gekennzeichnet, dass** Partner 1 des spezifischen Bindungspaares 1 Avidin oder Streptavidin ist.

4. Verfahren gemäß Anspruch 1, **dadurch gekennzeichnet, dass** Partner 1 des spezifischen Bindungspaares 2 ein Antikörper gegen Partner 2 des spezifischen Bindungspaares 2 ist.

5. Verfahren gemäß Anspruch 4, **dadurch gekennzeichnet, dass** Partner 1 des spezifischen Bindungspaares 2 ein Antikörper gegen Digoxigenin oder Digoxin ist.

6. Verfahren gemäß einem der Ansprüche 1 bis 5, **dadurch gekennzeichnet, dass** für die Herstellung der von Analyt abgeleiteten und diesen repräsentierenden Substanz Analyt mit Antikörpern versetzt wird, die an den Analyt binden, wobei ein Teil der Antikörper Partner 2 des spezifischen Bindungspaares 1 und der andere Teil der Antikörper Partner 2 des spezifischen Bindungspaares 2 trägt.

7. Verfahren gemäß einem der Ansprüche 1bis 5, **dadurch gekennzeichnet, dass** für die Herstellung der von Analyt abgeleiteten und diesen repräsentierenden Substanz Analyt mit Antigenen, Haptenen oder Oligopeptiden versetzt wird, wobei ein Teil der Antigene, Haptene oder Oligopeptide Partner 2 des spezifischen Bindungspaares 1 und der andere Teil der Antigene, Haptene oder Oligopeptide Partner 2 des spezifischen Bindungspaares 2 trägt.

8. Verfahren gemäß einem der Ansprüche 1bis 5, **dadurch gekennzeichnet, dass** der Analyt eine Nukleinsäure ist, die vervielfältigt wird, hierbei Partner 2 des spezifischen Bindungspaares 1 oder Partner 2 des spezifischen Bindungspaares 2 gebunden an ein Nukleotid oder ein Oligonukleotid in die Kopie der Nukleinsäure eingebaut und das Amplifikationsprodukt mit Nukleinsäure hybridisiert wird, die Partner 2 desjenigen Bindungspaares trägt, den die komplementäre Nukleinsäure nicht aufweist.

9. Verfahren gemäß einem der Ansprüche 1 bis 5, **dadurch gekennzeichnet, dass** der Analyt eine Nukleinsäure ist, die mit 2 Nukleinsäuresonden hybridisiert wird, wobei die eine Partner 2 des spezifischen Bindungspaares 1 und die andere Partner 2 des spezifischen Bindungspaares 2 enthält.

## Claims

1. Method for determining an analyte by means of specific binding pairs using an analytical element containing in or on material which enables liquid transport between zones, a sample application zone and a detection zone located downstream thereof, wherein the detection zone contains partner 1 of a specific binding pair 1 immobilized in such a manner that it is able to bind to partner 2 of the specific binding pair 1 which is not the analyte when it contacts it, and a labelled partner 1 of a specific binding pair 2 upstream of the detection zone which is present impregnated on a material such that it can be detached by liquid and is able to bind to partner 2 of the specific binding pair 2 which is not the analyte when this contacts it,
**characterized in that**
a substance derived from and representing the analyte which comprises partner 2 of the specific binding pair 1 and partner 2 of the specific binding pair 2 is contacted with the labelled partner of the specific binding pair 2 in the analytical element, is moved by liquid transport in the analytical element towards the detection zone that is located downstream of the sample application zone, is bound in the detection zone to partner 1 of the specific binding pair 1 and is determined on the basis of the label of partner 1 of the specific binding pair 2.

2. Method according to claim 1, **characterized in that** partners that belong together of specific binding pairs are selected from the group comprising hapten and antibody, antigen and antibody, lectin and sugar / saccharide, ligand and receptor, avidin / streptavidin and biotin, nucleic acid and nucleic acid.

3. Method according to claim 1, **characterized in that** partner 1 of the specific binding pair 1 is avidin or streptavidin.

4. Method according to claim 1, **characterized in that** partner 1 of the specific binding pair 2 is an antibody against partner 2 of the specific binding pair 2.

5. Method according to claim 4, **characterized in that** partner 1 of the specific binding pair 2 is an antibody against digoxigenin or digoxin.

6. Method according to one of the claims 1 to 5, **characterized in that** antibodies that bind to the analyte are added to the analyte where one part of the antibodies carries partner 2 or the specific binding pair 1 and the other part of the antibodies carries partner 2 of the specific binding pair 2 in order to prepare the substance derived from and representing the analyte.

7. Method according to one of the claims 1 to 5, **characterized in that** antigens, haptens or oligopeptides are added to the analyte where one part of the antigens, haptens or oligopeptides carries partner 2 of the specific binding pair 1 and the other part of the antigens, haptens or oligopeptides carries partner 2 of the specific binding pair 2, in order to prepare the substance derived from or representing the analyte.

8. Method according to one of the claims 1 to 5, **characterized in that** the analyte is a nucleic acid which is amplified whereby partner 2 of the specific binding pair 1 or partner 2 of the specific binding pair 2 bound to a nucleotide or to an oligonucleotide is incorporated into the copy of the nucleic acid and the amplification product is hybridized with a nucleic acid which carries partner 2 of that binding pair which the complementary nucleic acid does not have.

9. Method according to one of the claims 1 to 5, **characterized in that** the analyte is a nucleic acid which is hybridized with 2 nucleic acid probes one of which contains partner 2 of the specific binding pair 1 and the other contains partner 2 of the specific binding pair 2.

## Revendications

1. Procédé pour déterminer un analyte au moyen de paires de liaison spécifiques en employant un élément d'analyse contenant dans ou sur une matière permettant un transport de liquide entre zones, une zone d'application d'échantillon et une zone de détection placée en aval de celle-ci, la zone de détection contenant un partenaire 1 d'une paire de liaison spécifique 1, immobilisé de telle sorte qu'il est en mesure d'engager une liaison avec le partenaire de la paire de liaison spécifique 1, qui n'est pas un analyte, lorsqu'il vient en contact avec celui-ci, et qu'en amont de la zone de détection, un partenaire 1 marqué d'une paire de liaison spécifique 2 existe imprégné sur une matière, séparable à l'aide d'un liquide, et qu'il est en mesure de s'engager dans une liaison avec le partenaire 2 de la paire de liaison spécifique 2 qui n'est pas l'analyte,
**caractérisé en ce que**,
une substance dérivée de l'analyte et représentant celui-ci, qui contient le partenaire 2 de la paire de liaison spécifique 1 et le partenaire 2 de la paire de liaison spécifique 2, est amenée en contact avec le partenaire marqué de la paire de liaison spécifique 2 dans l'élément d'analyse, qu'elle se déplace par transport de liquide dans l'élément d'analyse en direction de la zone de détection placé en aval de la zone d'application d'échantillon, qu'elle se lie au partenaire 1 de la paire de liaison spécifique 1 dans la zone de détection et qu'elle est déterminée à l'aide du marquage du partenaire 1 de la paire de liaison spécifique 2.

2. Procédé selon la revendication 1, **caractérisé en ce que** les partenaires allant ensemble des paires de liaison spécifiques sont choisis dans le groupe formé par un haptène et un anticorps, un antigène et un anticorps, une lectine et un sucre/saccharide, un ligand et un récepteur, l'avidine/streptavidine et la biotine, un acide nucléique et un acide nucléique.

3. Procédé selon la revendication 1, **caractérisé en ce que** le partenaire 1 de la paire de liaison spécifique 1 est l'avidine ou la streptavidine.

4. Procédé selon la revendication 1, **caractérisé en ce que** le partenaire 1 de la paire de liaison spécifique 2 est un anticorps dirigé contre le partenaire 2 de la paire de liaison spécifique 2.

5. Procédé selon la revendication 4, **caractérisé en ce que** le partenaire 1 de la paire de liaison spécifique 2 est un anticorps dirigé contre la digoxigénine ou la digoxine.

6. Procédé selon l'une quelconque des revendications 1 à 5, **caractérisé en ce que**, pour préparer la substance dérivée de l'analyte et représentant celui-ci, l'analyte est mélangé à des anticorps qui se lient à l'analyte, une partie des anticorps portant le partenaire 2 de la paire de liaison spécifique 1 et l'autre partie des anticorps portant le partenaire 2 de la paire de liaison spécifique 2.

7. Procédé selon l'une quelconque des revendications 1 à 5, **caractérisé en ce que**, pour la préparation de la substance dérivée de l'analyte et représentant celui-ci, l'analyte est mélangé à des antigènes, des haptènes, ou des oligopeptides, une partie des antigènes, des haptènes ou des oligopeptides portant le partenaire 2 de la paire de liaison spécifique 1 et l'autre partie des antigènes, des haptènes ou des oligopeptides portant le partenaire 2 de la paire de liaison spécifique 2.

8. Procédé selon l'une quelconque des revendications 1 à 5, **caractérisé en ce que** l'analyte est un acide nucléique qui est reproduit, ici le partenaire 2 de la paire de liaison spécifique 1 ou le partenaire 2 de la paire de liaison spécifique 2 sont liés à un nucléotide ou à un oligonucléotide dans la copie de l'acide nucléique, et le produit d'amplification s'hybride avec l'acide nucléique, qui porte le partenaire 2 de cette paire de liaison, que l'acide nucléique complémentaire ne présente pas.

9. Procédé selon l'une quelconque des revendications 1 à 5, **caractérisé en ce que** l'analyte est un acide nucléique, qui s'hybride avec 2 sondes à base d'acide nucléique, l'une contenant le partenaire 2 de la paire de liaison spécifique 1 et l'autre contenant le partenaire 2 de la paire de liaison spécifique 2.
